# EUROPEAN PATENT SPECIFICATION

(11) **EP 2 030 566 B1**
(45) Date of publication and mention of the grant of the patent: **24.08.2016**
(21) Application number: 07017058.4
(22) Date of filing: 31.08.2007
(51) Int. Cl.: A61B 5/15

(54) **Analysis system for determining an analyte in a body fluid, magazine for an analysis system and analyzing element, and method for analyzing a body fluid**
Analysesystem zur Bestimmung eines Analyten in einer Körperflüssigkeit, Behälter für ein Analysesystem sowie Verfahren zur Analyse einer Körperflüssigkeit
Système d'analyse pour déterminer une analyse dans un fluide corporel, magazine pour système d'analyse et procédé d'analyse d'un liquide corporel

(43) Date of publication of application: 04.03.2009
(60) Divisional application: 13160311.0
(73) Proprietor: Roche Diabetes Care GmbH, 68305 Mannheim (DE); F.Hoffmann-La Roche AG, 4070 Basel (CH)
(72) Inventor: List, Hans, 64754 Hesseneck-Kailbach (DE); Frey, Stephan-Michael, 64347 Griesheim (DE); Flügge, Kai, 52070 Aachen (DE)
(74) Representative: Petirsch, Markus

(56) References cited:
- WO-A-2005/018711
- WO-A-2005/104949
- WO-A-2006/031920
- WO-A-2006/038044
- US-A1- 2004 092 995
- US-A1- 2006 229 532
- US-A1- 2007 149 897

## Description

The present invention relates to an analysis system for determining an analyte in a body fluid comprising a reusable analysis instrument having a drive and a measuring and evaluation unit for measuring a measurement variable characteristic for the determination of an analyte and a magazine having a plurality of flat, disposable, integrated sample acquisition and analyzing elements, which each include a puncturing element and an analyzing element and have two major sides. The present invention also relates to a magazine for an analysis instrument having a plurality of flat, elongate, disposable, integrated sample acquisition and analyzing elements as well as the integrated sample acquisition and analyzing element itself, which comprises an analyzing element and a puncturing element having a tip. The puncturing element is movable on a movement path of a puncture movement in the puncturing direction and in relation to the analyzing element. In addition, the present invention relates to a method for generating a wound in a body part for analyzing a body fluid exiting from the wound by means of an analysis system, which comprises a reusable analysis instrument and a magazine having a plurality of flat, disposable, integrated sample acquisition and analyzing elements.

To determine an analyte in a body fluid for diagnostic purposes, small quantities of this fluid are taken from a body part. Frequently, blood is analyzed as a body fluid for this purpose. Puncturing devices having a lancet are used for the purpose of generating a wound in a body part, such as the fingertip or the earlobe. Puncturing devices of this type are implemented in such a manner that they can be used not only by medical personnel, but also by medical laypersons.

A plurality of steps must be performed to determine an analyte in the body fluid. Firstly, a wound must be generated in the body part, from which a body fluid can exit; in addition, the body fluid must be analyzed using a measuring unit. This can be performed using two devices operating independently from one another. The use of two devices is quite uncomfortable for the user. Therefore, combined systems are frequently used, which both generate the wound and also analyze the exiting fluid.

In addition to simple operation and increased operating comfort, a compact design of the devices is therefore necessary. In addition, piercing which is as pain-free as possible is required. This increases the acceptance of devices of this type for diabetics in particular, who have to determine the glucose content in their blood a plurality of times a day.

An integrated analysis instrument having a test sensor and a lancet is known, for example, from WO 2006/092281, which determines an analyte in a body fluid by electrochemical measurement. A capillary channel is provided in a test strip, via which a received body fluid is transferred to test electrodes, to determine an analyte by means of a measuring unit. The lancet is located on the bottom side of the test strip. The lancet is movable in relation to the test strip and is enclosed by a sterile envelope, from which it exits before piercing into the body part. The body fluid exiting from the wound is suctioned in by the capillary channel on the top side of the test strip and conducted to the electrodes of the electrochemical measuring unit.

Because of the coupling mechanism of the analysis instrument to the integrated test sensor, the instrument is used with individual test sensors. Locating a plurality of disposable test sensors in a magazine is not provided.

To avoid complex use of individual integrated puncture and analyzing elements, analysis systems are known in which the puncture and analysis elements are stored in a magazine. Such a system is described, for example, in US 2003/0212345. The puncture and analyzing element used comprises a needle having a capillary channel, which is molded onto an analyzing element. Blood acquired during piercing is conducted through the capillary channel to the test zone of the puncture and analyzing element, which is implemented in one piece. The used elements are stored in a separate magazine or ejected from the system after use.

A plurality of systems are known in the prior art, in which integrated sensors having a puncturing element and an analyzing element are located in a magazine as for example US 2004/0092995, WO 2006/031920 and WO 2005/104949. US 6,607,658, EP 1402818, and US 2002/0052618 disclose sensors of this type, for example. However, the sensors have to be completely removed from the magazine to be able to perform their function. This requires a relatively large construction of the analysis instruments.

An analysis system having an integrated puncture and analyzing element which is stored in a magazine is disclosed in US 2003/0143113. The puncture and analyzing element is not completely removed from the magazine, which simplifies the automated handling in the analysis system, in particular if the puncture and analysis system is moved back in the magazine after use. Because the puncture and analyzing element is implemented in one piece, the test zone of the analyzing element is also subjected to a sterilization of the puncturing element, so that the analysis capability of the test zone is reduced.

It is therefore an object of the present invention to suggest a compact analysis system having improved properties, in particular having a small overall size, in which a plurality of integrated sample acquisition and analyzing elements are stored.

The present object is achieved by an analysis system having the features of claim land a magazine having the features of claim 5. A method having the features of claim 18 is specified to achieve the stated object.

The analysis system according to the present invention for determining an analyte in a body fluid comprises a reusable analysis instrument and a magazine having a plurality of flat, disposable, integrated sample acquisition and analyzing elements, which each include a puncturing element and an analyzing element and have two major surfaces. These integrated sample acquisition and analyzing elements are designated in professional circles as "disposables". The term "disposables" or "dispos" is used in the following.

The analysis instrument has a drive for driving the puncture movement of the puncturing element, a coupling unit for coupling a disposable to the drive, and a measuring and evaluation unit for measuring a measurement variable, which is characteristic for the determination of an analyte, and for deriving a desired analysis result. The analysis instrument includes a holder for holding the magazine. The magazine comprises a housing having a plurality of elongate chambers located neighboring one another, which are separated by two side walls running in the longitudinal direction of the chambers. The height and length of the side walls is greater than its width, which is defined by the distance of two neighboring side walls to one another. The chambers have major sides delimited by the side walls and two long and two short minor sides extending between the side walls. One chamber can accommodate one disposable. Each chamber has an outlet opening on one of its minor sides, through which a puncturing element of the disposable located In the chamber, which is moved on the movement path, and which is movable in the puncturing direction, can exit at least partially from the chamber.

The coupling unit of the analysis instrument has a coupling element; the puncturing element has a coupling structure which corresponds to the coupling element. The chambers of the magazine are accessible for the coupling element of the coupling unit on one of their long minor sides in such a manner that the coupling element can be formfitting coupled to the coupling structure of the puncturing element when the puncturing element is positioned in the chamber of the magazine and the magazine is located in the holder of the analysis instrument. The coupling unit of the analysis instrument has another coupling element that can be coupled to a coupling structure of the analyzing element of the disposable when the analyzing element is positioned in the chamber of the magazine.

One advantage of the analysis system according to the present invention is the compact construction, which allows a close configuration of the individual disposables to one another. Two neighboring disposables are only separated by a side wall between two chambers. In addition, the analysis system according to the present invention offers good and simple access to the disposable, in particular to the puncturing element, to drive it on its movement path during the puncture movement.

The accommodation of the individual disposables in one chamber each allows individual storage of the disposables. Therefore, not only the unused, new disposable can be stored in the magazine, but rather the disposable can also be returned back into the chamber after its use. Contamination of the still unused neighboring disposables in the magazine is precluded by the individual storage.

The magazine according to the present invention is insertable into the holder of the analysis instrument. Preferably, a magazine of this type comprises between 10 and 50 disposables, especially preferably 25 disposables. The content of the magazine is sufficient for several days. The user does not have to insert a new disposable into the analysis system after each puncture and analysis procedure. A new disposable is automatically available before each puncture procedure.

The magazine according to the present invention has the advantage that due of the edgewise positioning of the disposables, the individual parts can be stacked densely, so that especially space-saving storage of the disposables is possible. Nonetheless, the disposables can be removed individually from the magazine, because the coupling for their activation is performed from the long minor side. It is obvious for a person skilled in the art that also a flat positioning of the disposables one above the other leads to the advantage of a space-saving storage and an individual activation and that such a flat positioning is included in the scope of the invention.

To further reduce the danger of contamination of a still unused disposable by an already used disposable, the major sides of the individual chambers delimited by the side walls are preferably closed. The minor sides of the chambers can also be at least partially closed. In a preferred embodiment, all sides of the chambers are closed. The long minor side, on which the chamber is accessible for the coupling element of the coupling unit, and the short minor side, on which the exit opening is located, are covered by a film. The still unused disposables are therefore enclosed completely hermetically sealed in a chamber of the magazine, which not only precludes the risk of a contamination. The possibility also arises of keeping the disposables dry easily. In particular, a test field zone provided on the analyzing element and having reagents, which are used for determining the analyte, can be kept dry reliably and effectively.

The film for covering the chambers of the magazine can be a thin layer, a coating or a sheet especially of plastic or a foil or the like. The film in this sense is adapted to be opened by or slit from the puncturing element and/or a coupling element of the coupling unit and/or another component of the analysis instrument. The film for covering the long minor sides of the chambers is typically a one-piece film. It preferably covers the magazine completely on the side on which the long minor sides of the chambers, through which the chambers are accessible, are located. This side of the magazine is typically the top side. The film is attached to the front faces of the side walls forming the major sides, for example, the film is glued on.

additionally or alternatively, the exit openings of the chambers are also covered by a film. The film is preferably in one piece and extends over the entire side of the magazine on which the exit openings of the chambers are located. The film can be glued or welded onto the front sides of the side walls.

In a preferred embodiment, the film for covering the minor sides of the chambers is implemented as at least partially metallic. It preferably comprises metal, for example, aluminum or an aluminum alloy. The film can also comprise a metallically coated or metallically vapor-deposited plastic. In particular metal films have the advantage that they are easy to pierce and separate. Uncontrolled further tearing of the film is excluded. The seal of the neighboring chambers is not unintentionally destroyed.

Another magazine according to the present invention has a plurality of disposables, each comprising a puncturing element and an analysing element. The magazine comprises a plurality of chambers located neighbouring one another.

Each chamber can contain a disposable. The puncturing element of the disposable is enclosed by a protective envelope. This has the advantage that the puncturing element cannot get into contact with the analysing element of the disposable or a reagent complement of the analysing element.

In addition, the chambers are covered with a film (on at least one of their sides), such that the chambers are completely closed. The chambers are enveloped so that they are hermetically sealed against the environment. Therefore, the disposable, especially the analysing element of the disposable, can easily be kept dry in the chamber such that no humidity or fluid can reach the inside of the chambers.

This magazine is also insertable into the holder of the analysis system according to the invention. The advantages of the magazin correspond to the advantages of the other magazine according to the invention.

The chambers of this magazine have the same or an equal geometry as the chambers of the first magazine according to the invention.

In a preferred embodiment of the magazine, the chambers are accessible on one of their long minor sides, such that a coupling element of a coupling unit can be coupled to the disposable. Preferably, at least the long minor sides, on which the chambers are accessible, are covered with a film.

In a preferred embodiment, the disposable which is stored in the magazine according to the present invention and used in the analysis system according to the present invention, is implemented in such a manner that the analyzing element has a test field zone. The body fluid to be analyzed is placed on this test field zone. The body fluid is typically blood, which is described as a special example in the following without restriction of the generality. The analyte to be determined is usually glucose in the case of blood transfer. However, other components of the blood and/or the body fluid can be analyzed, such as lactates or similar substances.

The puncturing element of the disposable preferably has a capillary channel having at least one sample inlet and one sample outlet. The movement path of the puncturing element comprises a transfer position, in which the puncturing element is positioned in relation to the analyzing element in such a position that the sample outlet of the capillary channel neighbors the test field zone of the analyzing element. The sample outlet is located in relation to the test field zone in such a manner that a transfer of a body fluid from the capillary channel onto the test field zone can take place. A disposable of this type having a puncturing element having a capillary channel is described in greater detail in EP 07005222.0; its content is made part of the content of this application by reference.

According to the present invention, the integrated sample acquisition and analyzing element (disposable) has two major sides which are preferably diametrically opposite flat sides. The disposable comprises an analyzing element and a puncturing element having a tip. The puncturing element is movable on the movement path of a puncture movement in the puncturing direction and in relation to the analyzing element at the same time. It is enclosed by a protective envelope which is produced from plastic or a thin plastic film. The protective envelope is connected to the puncturing element and the analyzing element. Upon a relative movement of the puncturing element to the analyzing element in the puncturing direction, the protective envelope is telescoped. The tip of the puncturing element penetrates the protective envelope and extends out of the protective envelope.

The protective envelope is advantageously connected to the rear end of the puncturing element in the puncturing direction, i.e., the end diametrically opposite the tip, for example, glued on. The tip of the puncturing element is mounted loosely in the protective envelope. This ensures that the tip does not unintentionally destroy the protective envelope. The front area of the protective envelope, which is near the tip of the puncturing element, is connected, preferably glued, to the analyzing element.

The protective envelope of the disposable does not have to be opened or slit from a component of the analysis instrument. The forces to be applied by the component of the analysis instrument are less in this type of disposable. The analysis instrument can therefore be implemented more simply, which results in lower production costs.

The disposable has a puncturing element which has a coupling structure. A coupling unit of an analysis instrument can be coupled to the coupling structure to move the puncturing element on its movement path. The coupling element advantageously corresponds to the coupling structure of the puncturing element in such a manner that a formfitting coupling is produced between the coupling element and the coupling structure. Preferably, the coupling structure of the puncturing element is located on a minor side or at the edge or edge side of the puncturing element, such that the coupling element can couple to the puncturing element from a direction traverse to the puncturing direction. Especially the coupling movement is in the same plane as the puncturing element.

In a preferred embodiment, the puncturing element comprises a needle element and an engagement element. The engagement element, which is also called a retention element or carrier, preferably includes the coupling structure of the puncturing element. The carrier is coupled or connected to the needle element. For example, the carrier can be glued externally onto the protective envelope. In the meaning of the present invention, the element and/or elements which move during puncturing and execute a puncturing movement on a movement path are considered the puncturing element. The puncturing element can therefore also comprise further elements, in particular the carrier.

To generate a wound in a fingertip for analyzing blood exiting from the wound, a method having a plurality of steps is executed according to the present invention. The method is performed by an analysis system which has at least a reusable analysis instrument and a magazine having a plurality of flat disposables. The disposables each include a puncturing element and an analyzing element. The analysis instrument has a drive and a coupling unit having a coupling element for coupling the analyzing element of the disposable to the drive. A measuring and evaluation unit and a holder for receiving the magazine are also components of the analysis instrument.

The magazine has a plurality of (elongate) chambers located neighboring one another, which each can receive one disposable. The chambers can be arranged all in one rigid body, positioned parallel to each other in form of a linear array, or in form of a drum with the chambers alongside the cylindrical outer surface, or can be arranged radially or semi radially in a disc shaped body, or can even be single chambers, movably connected to form a belt or a chain. The most compact arrangement is a linear array.

The analyzing element of the disposable has a test field zone in which the reagents for determining the analyte are preferably contained, and a coupling structure. The chambers of the magazine in which the disposable is stored are accessible in such a manner that the coupling element of the coupling unit can formfitting couple to the analyzing element.

The method comprises at least the following steps: firstly, the coupling element of the coupling unit is coupled in a formfitting way to the coupling structure of the analyzing element. Subsequently, the analyzing element is moved in the puncturing direction into an operational position, in which the analyzing element extends out of the chamber of the magazine so that the test field zone of the analyzing element is positioned outside the magazine. In a further step, a measurement variable which is characteristic for the determination of the analyte is measured on the test field zone.

To measure the measurement variable, the test field zone is located in the operational position outside the magazine. Preferably, an optical measurement of the measurement variable is performed; an electrochemical measurement is also possible. The measurement optic is advantageously located directly in front of the magazine. At least a part of the disposable is preferably located in the chamber of the magazine, so that the chamber can be used as a guide and holder for the disposable. Because the disposable is guided by the chamber during its movement into the operational position and as it remains in this position, the mechanism of the analysis instrument for moving the disposable is significantly simpler than in systems in which the disposable is moved completely out of the magazine.

The method further comprises the following: the coupling unit has a second coupling element and the puncturing element also has a coupling structure. Further, the puncturing element preferably comprises a capillary channel having at least one sample inlet and one sample outlet. The capillary channel is preferably located on a needle element of the puncturing element. The coupling structure can be provided on the needle element itself or on a carrier connected to the needle element of the puncturing element, as described above.

In a further method step, the second coupling element is formfitting coupled to the coupling structure of the puncturing element. The puncturing element is moved in relation to the analyzing element on the movement path in the puncturing direction in such a position that the tip of the puncturing element projects beyond the analyzing element in the puncturing direction. A wound is generated in a body part located in the puncturing direction in front of the analyzing element and a body fluid from the wound is received to the sample inlet in the capillary channel of the puncturing element. This step is preferably performed when the analyzing element is already located in the operational position. Furthermore, the puncturing element is moved relative to the analyzing element into a transfer position in such a manner that the sample outlet of the capillary channel of the puncturing element neighbors the test field zone of the analyzing element. The puncturing element and the analyzing element do not come into contact before the next step is performed. In a further step, the puncturing element and the analyzing element can contact one another, a contact pressure unit of the analysis instrument preferably pressing the sample outlet of the capillary channel against the test field zone.

The present invention is explained in greater detail in the following on the basis of preferred embodiments without restriction of the generality. The special features illustrated therein can be used individually or in combination to provide preferred embodiments of the present invention.
- Figure 1: shows a schematic illustration of the analysis system according to the present invention;
- Figure 2: shows a magazine having a plurality of chambers;
- Figure 3: shows a detail view of the integrated sample acquisition and analyzing element according to the present invention;
- Figures 4a to e: show a detail view of the analysis system during the puncture procedure in different movement phases, and
- Figure 5: shows a detail view of the analysis system during the determination of an analyte in a body fluid

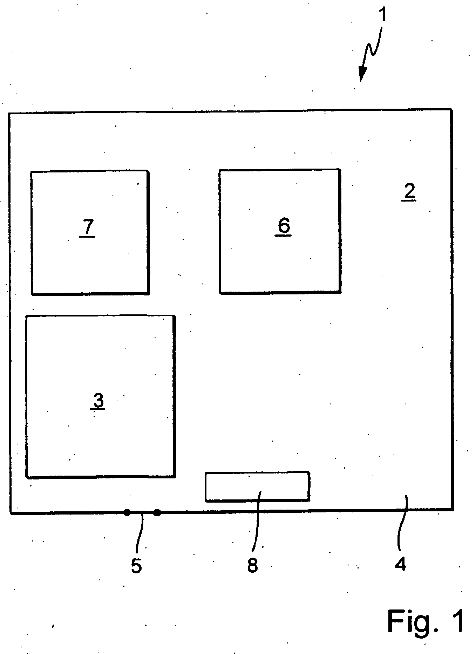

Figure 1 schematically shows an analysis system 1, which comprises an analysis instrument 2, into which a magazine 3 is inserted. The analysis instrument 2 has a housing 4 having a housing opening 5, through which a disposable 15 or puncturing element stored in the magazine 3 can exit from the housing 4.

The analysis instrument 2 comprises a drive 6, a coupling unit 7, and a measuring and evaluation unit 8. The analysis instrument 2 also includes further components (not shown), such as a holder for the magazine 3.

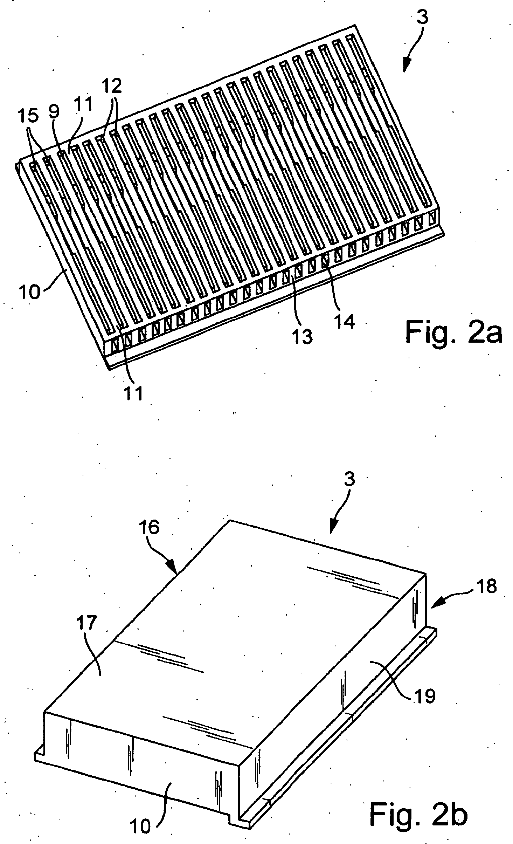

The magazine 3 is shown in detail in Figures 2a and b. It has a housing 3a having a plurality of chambers 9 located neighboring one another. The chambers 9 are implemented as elongate slots or grooves in the exemplary embodiment shown. A side wall 10 running in the longitudinal direction, which separates two chambers 9 from one another, is located between each two neighboring chambers 9. The height and length of the side walls 10 are greater than the distance between them. In this way, narrow, elongate chambers 9 are formed, which each have two major sides 11 delimited by the side walls 10 and two long minor sides 12 and two short minor sides 13 extending between the major sides 11.

It can be seen from Figure 2a that the chambers 9 are open on their long minor side 12, which is directed upward in the figure. The short minor sides 13 visible in the perspective illustration have an exit opening 14, through which a disposable 15 located in a chamber 9 can exit. The long minor sides 12 and the side walls 10 and major sides 11 thus extend in the puncturing direction.

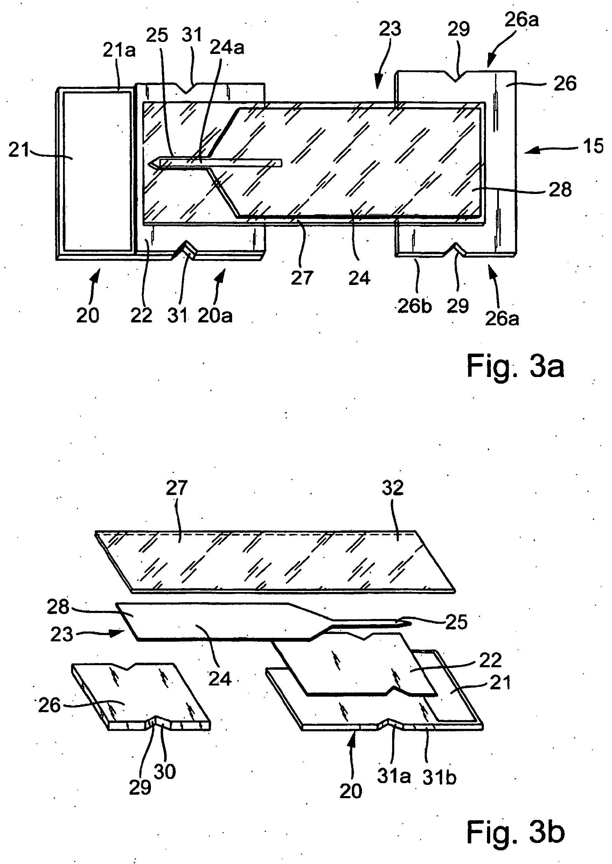

Figure 2b shows the magazine 3 from Figure 3, in which the top side 16 is covered by a protective film 17. The protective film 17 closes the upwardly directed long minor sides 12 of the chambers 9. It is glued or welded onto the narrow, upwardly directed front sides of the side walls 10.

The short minor sides 13 of the chambers 9, which have an exit opening 14 here, are located on a front side 18 of the magazine 3. The front side 18 is covered by a protective film 19 (like the top side 16). The protective film 19 is glued or welded to the short front sides of the side walls 10. During the puncturing or analysis procedure, the disposable 15 passes at least partially through the exit opening 14 and penetrates the film 19. Alternatively, the protective film 17 and the protective film 19 can be formed by a single film. The protective films 17 and 19 allow the individual chambers 9 of the magazine 3 to be completely enclosed and hermetically sealed. A disposable 15 located in one chamber 9 is thus encapsulated in relation the environment. This is particularly important for keeping the disposables dry. The protective films 17, 19 are preferably films impermeable to water vapor, so that the unused disposable 15 in a chamber 9 can be kept dry. For this purpose a desiccant (drying agent) is additionally placed in the chamber 9. In a preferred embodiment, the disposable 15 comprises the desiccant (e.g., in form of a so-called carrier). A further desiccant then does not have to be placed in the chamber.

Figure 3a shows an embodiment of a disposable 15 according to the present invention, Figure 3b shows an exploded view of the disposable 15 from Figure 3a.

The disposable 15 comprises an analyzing element 20 and a puncturing element 23. The analyzing element 20 has a test field zone 21 and a spacer element 22 on one of its top sides 21 a, the spacer element 22 being located behind the test field zone 21 in the puncturing direction. The spacer element 22 is glued onto the top side 21 a, but can also be attached in other ways. It is used as a spacer between the puncturing element 23 and the analyzing element 20, so that the puncturing element 23 does not contact the test field zone 21 on its puncturing path. The configuration of a disposable 15 having a spacer element 20 between an analyzing element and a puncturing element is described in detail in EP 07005222.0.

The puncturing element 23 comprises a needle element 24 having a needle tip 25 and a carrier 26. The needle element 24 has a capillary channel 24a, which extends opposite to the puncturing direction from the needle tip 25. The capillary channel 24a is located on the side facing toward the analyzing element 20 and is implemented as a half-open groove, for example. The needle element 24 is completely enclosed by a protective envelope 27. The protective envelope 27 is glued to the needle element 24 on its rear end 28, which is diametrically opposite the needle tip 25. In the area of the needle tip 25, the protective envelope 27 loosely encloses the needle tip 25, so that it is movable in the protective envelope. The outside of the protective envelope 27 is also glued onto the carrier 26 in the rear area 28 of the needle element 24. Thus, the carrier 26 and the needle element 24 are coupled and connected to one another (via the protective envelope 27).

The carrier 26 is preferably formed by a film or a plastic part, whose thickness is less than 1 mm. For example, the carrier 26 can also contain the desiccant to keep dry the unused disposable 15 in the closed chamber 9. The carrier 26 has a coupling structure 29 on each of its lateral edges 26a on the minor sides 26b, which extend in the puncturing direction. The coupling structure 29 is implemented as a recess in the lateral delimitation profile of the carrier 26. The recess can also be implemented as an indentation or undercut or as a hole located in the boundary area in one of the flat sides of the carrier. It can also be formed by a pin or a projecting element. It is only important for this purpose that the coupling structure 29 can be coupled to a corresponding coupling element of the coupling unit 7 of the analysis instrument 2 in a formfitting way. Therefore, it is possible that a coupling structure 29 (e.g. a recess) is located on only one minor side 26b.

In an alternative embodiment, the coupling structure 29 can be located directly at the needle element 24 of the puncturing element 23 if no carrier 26 is used. Formfitting coupling between the coupling structure 29 in the needle element 24 and a coupling element of the coupling unit can take place through the (thin) protective envelope 27, in other words with the protective envelope 27 in between.

The analyzing element 20 also has a coupling structure 31, to which a further coupling element of the coupling unit 7 can couple, preferably in a formfitting way. The coupling structure 31 of the analyzing element 20 is implemented on the boundary area 20a. It can be a recess (shown), a pin, a hook, a hole, an oblong hole, or a similar feature. The coupling structure 31 comprises a coupling structure 31 a on the analyzing element 20 and a corresponding coupling structure 31 b on the connected spacer element 22 of the analyzing element 20. Preferably, the coupling structures 29,31 are in the same plane and are accessible from the same direction.

During the puncturing movement, the puncturing element 23 is moved in relation to the analyzing element 20, the carrier 26 being moved up to the analyzing element 20. The protective envelope 27, which is connected in its front area 32 to the analyzing element 20 and/or the spacer element 22, and at its rear end 28 to the needle element 24, is telescoped during the puncture movement of the puncturing element 23 like an accordion. This causes the needle tip 25 to penetrate the protective envelope 27 and to exit out of it.

In a preferred embodiment, the coupling structures 29 and 31 are each located on the same spatial direction of the disposable 15, so that coupling to a coupling unit can take place from the same direction. The two coupling structures 29 and 31 are thus located in such a manner that, if the disposable 15 is stored in the magazine 3, a coupling unit can couple in each case from above through the top side 16 of the magazine 3. The coupling movement (for coupling the coupling unit to the coupling structures 29,31) is a motion transversal to the puncturing movement and preferably takes place in the same plane as the puncturing movement.

The disposable 15 according to the present invention has the advantage that during its production, firstly the needle element 24 of the puncturing element 23 can be hermetically enclosed by the protective envelope 27. Subsequently, the needle element 24 is sterilized, for example, by gamma or electron beams. In a next step, the analyzing element 20 is glued jointly with the spacer element 22 to the protective envelope 27. The carrier 26 is also connected to the protective envelope 27. Alternatively, the carrier 26 can also be mounted even before the sterilization of the puncturing element 23.

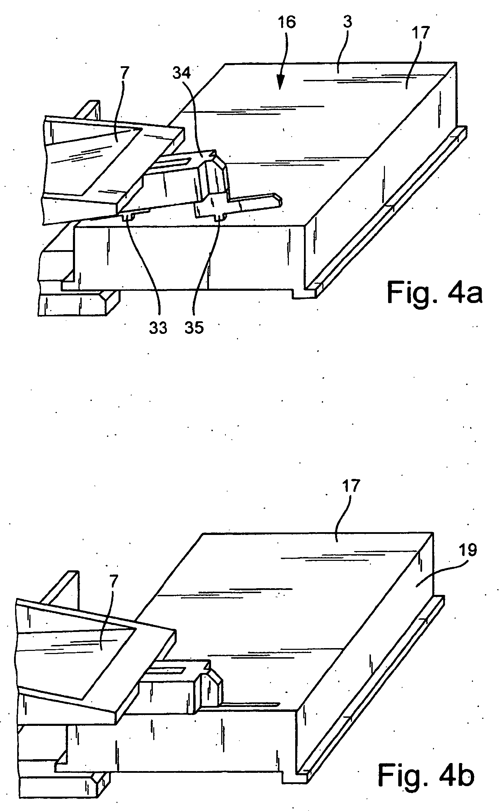

Figures 4a to e show the magazine 3 and a part of the coupling unit 7 during different movement phases of the disposable 15.

The coupling unit 7 comprises a coupling element 33 for coupling to the coupling structure 29 of the puncturing element 23. The coupling element 33 is located on an extension arm 34. A further coupling element 35 for coupling to the coupling structure 31 of the analyzing element 20 is also located on the extension arm 34. The two coupling elements 33, 35 can be moved synchronously or individually in relation to one another. Therefore, the puncturing element 23 and the analyzing element 20 can also be moved synchronously or individually and relative to another. In the exemplary embodiment shown here, the coupling elements 33, 35 are implemented as pins which ensure formfitting coupling to the coupling structure 29 and 31, respectively.

Figure 4b shows how the extension arm 34 of the coupling unit 7 has been lowered (in direction to the magazine 3), the coupling elements 33 and 35 having penetrated the protective film 17. The film 17 is only penetrated and destroyed in the area which covers the chamber 9, in which the disposable 15 to be used is stored. The coupling elements 33 and 35 are coupled in a formfitting way to the coupling structures 29 and 31, respectively.

The extension arm 34 of the coupling unit 7 moves in the puncturing direction, the two coupling elements 33 and 35 being moved synchronously with one another.

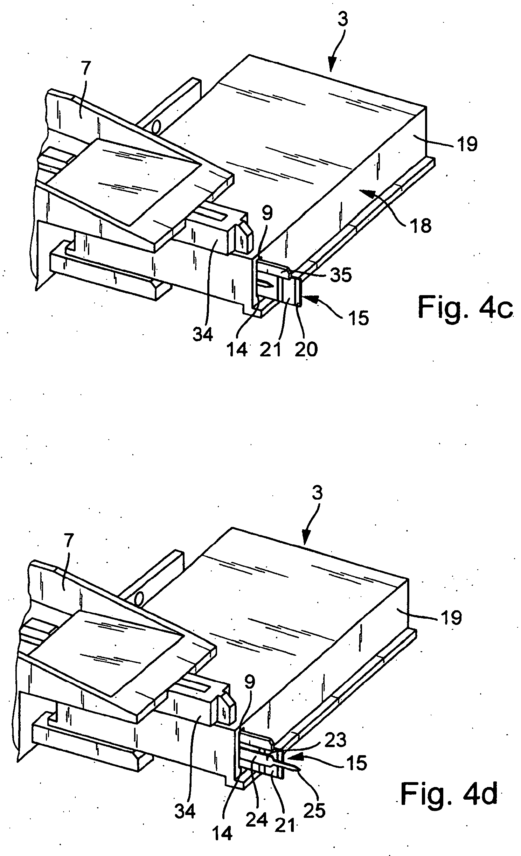

Figure 4c shows the coupling elements 33, 35 of the extension arm 34 moved in the puncturing direction until the disposable 15 has penetrated the protective film 19 on the front side 18 of the magazine 3. Preferably, the disposable 15 is moved so far into the puncturing direction that the test field zone 21 of the analyzing element 20 is located in front of an optical measuring apparatus of the measurement and evaluation unit 8. Alternatively, a relative movement of the two coupling elements 33, 35 to one another can be performed in such a manner that the needle tip 25 of the puncturing element 23 projects beyond the analyzing element 20 and the needle tip 25 penetrates the protective film 19.

Figure 4c shows the disposable 15 in its working position, in which the disposable 15 projects partially through the exit opening 14 and the test field zone 21 of the disposable 15 is located outside the magazine 3. Because a (large) part of the disposable 15 remains in the chamber 9 of the magazine 3, good guiding by the chamber 9 of the magazine 3 during the puncture movement and in the working position is ensured. It is sufficient that the coupling elements 33, 35 engage from above (from the top side 16 of the magazine 3) into the coupling structures 29, 31. Forces in and opposite to the puncturing direction must primarily be transmitted, but typically no transverse forces. The coupling elements 33, 35 can be implemented very narrow and the disposable 15 can be implemented very flat. In this way, many disposables 15 can be stored in the magazine 3 in a small space, which finally results in a small overall size of the analysis instrument 2.

In Figure 4d, the puncturing element 23 has executed a movement in relation to the analyzing element 20, so that the needle tip 25 projects beyond the analyzing element 20. In this position, a body part can be pierced to generate a wound.

While the needle tip 25 is located in the body part, blood is received in the capillary channel (not shown here) of the needle element 24. The capillary channel implemented as an open groove is preferably hydrophilic, so that blood is suctioned into the channel. Details of a puncturing element having a capillary channel are described extensively in the application EP 07005222, to which reference is made here.

After the puncturing, the puncturing element 23 is moved opposite to the puncturing direction by the coupling element 33 until the puncturing element 23 is located in a transfer position in relation to the analyzing element 20. In the transfer position, the sample outlet of the capillary channel neighbors the test field zone 21. The transfer position is thus a relative position of the puncturing element 23 to the analyzing element 20; the operational position, in contrast, is the relative position of the analyzing element 20 to the magazine 3 and/or to the chamber 9.

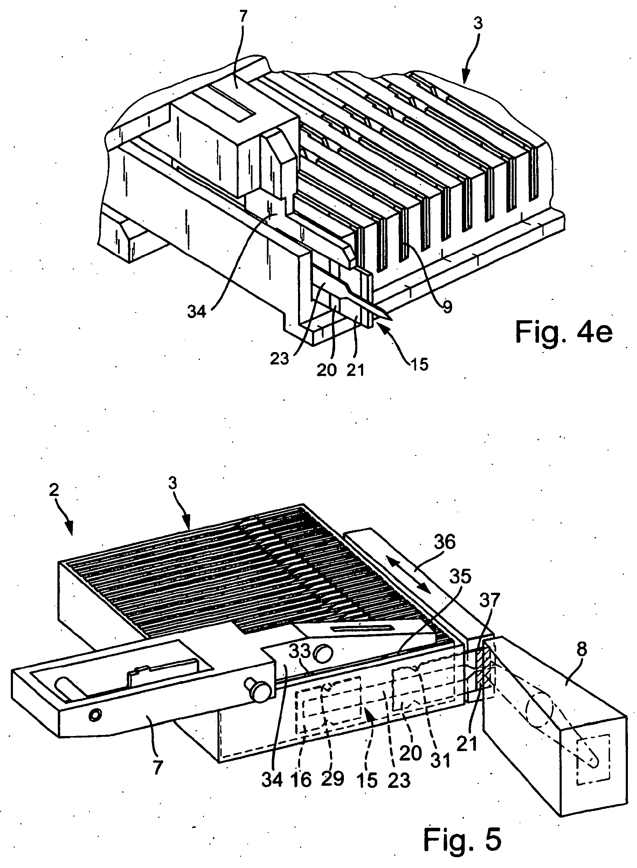

Figure 4e shows the magazine 3 (without protective film 17, 19) and the puncturing element 23 being in the transfer position. The body fluid can be transferred to the test field zone 21. The test field zone 21 preferably includes a test field which comprises at least one absorbent layer. The test field of the test field zone 21 has a receptacle surface, which is located on the top side of the analyzing element 21a facing toward the puncturing element 23. A detection surface 37 on the diametrically opposite bottom side of the analyzing element 20 contains a reagent system, typically comprising a plurality of components, whose reaction to the body fluid results in an optically measurable change of the detection surface 37.

To improve the sample transfer from the puncturing element 23 onto the test field zone 21, the puncturing element 23 and the analyzing element 20 are pressed against one another. This can be performed by a contact pressure unit, for example.

As shown in the figures 4a to 4e, the unused disposable 15 is in a first position (unused position). In this position, the puncturing element 23 has no contact to the analyzing element 20. On the movement path of the disposable 15 to generate a wound in a body part and to analyze a body fluid exiting from the wound, the disposable 15 is in a second position, in which the puncturing element 23 and the analyzing element 20 are in contact to another.

Preferably, as shown in figures 4a to 4e, the disposable 15 extends only partially out of the chamber 9, so that the rear end of the disposable 15 is contained in the chamber during the whole puncturing procedure. This means that especially the analyzing element 20 and the puncturing element 23 are guided by the chamber 9 during the puncturing procedure because both elements 20,23 extend only partially out of the chamber. Thus, the movement of the puncturing element 23 is very stable which results in a painless puncture of the finger tip.

Figure 5 shows a further detail view of the analysis instrument 2 according to the present invention having a magazine 3. Only the extension arm 34 and the two coupling elements 33, 35, which engage in the corresponding coupling structures 29, 31 of the disposable 15, are shown of the coupling unit 7. The optical measuring and evaluation unit 8 is located in front of the magazine 3 in the puncturing direction. The disposable 15 is located in the operational position such that the test field zone 21 is located outside the magazine 3. In the operational position the test field zone 21 is positioned in front of the optic of the optical measuring and evaluation unit 8.

In the embodiment shown here, the puncturing element 23 is located behind the analyzing element 20 in the plane of the drawing. The measuring and evaluation unit 8 positioned transversely (e. g. perpendicular) to the puncturing direction neighbors the bottom side of the analyzing element 20 and thus the detection surface 37, so that the distance between them is only a few millimeters. A contact pressure unit 36, which is also located in front of the magazine 3 in the puncturing direction, presses the puncturing element 23 against the analyzing element 20 to create contact between them and to improve the blood transfer. The contact pressure unit 36 can be implemented as a plunger, tappet, or the like, for example. During the contact pressure, the measurement and evaluation unit 8 can form a buttress to the contact pressure unit 36.

After the desired analyte has been determined in the blood, in a further method step, the disposable 15 is moved back out of the operational position opposite to the puncturing direction until it is again completely positioned in the chamber 9. Preferably, the puncturing element 23 and the analyzing element 20 are moved together, especially synchronically to another.

In the next step, the extension arm 34 of the coupling unit 7 can be moved upward, so that the coupling between the coupling elements 33, 35 and the coupling structures 29, 31 is disengaged. The magazine 3 can be moved transversely to the puncturing direction, so that upon the next use of the analysis system 1 according to the present invention, a sealed chamber 9 having an unused disposable 15 is located below the coupling elements 33, 35 of the coupling unit 7. The system is then ready for the next use.

## Claims

1. Analysis system for determining an analyte in a body fluid, the system comprising a reusable analysis instrument (2) and
a magazine (3) including a plurality of flat, disposable, integrated sample acquisition and analyzing elements, each comprising a puncturing element (23) and an analyzing element (20) and having two major surfaces,
the analysis instrument (2) comprising
a drive, by which a puncture movement of the puncturing element (23) is driven, for
movement on a movement path, the puncture movement including a propulsion phase in the puncturing direction and, after reaching a reversal point, a retraction phase opposite to the puncturing direction,
a coupling unit (7), adapted for coupling an integrated sample acquisition and
analyzing element to the drive,
a measuring and evaluation unit (8) for measuring a measurement variable
characteristic for the determination of an analyte and for deriving therefrom a desired analysis result, and
a magazine holder for holding the magazine (3),
the magazine (3) comprising
a plurality of elongate chambers (9) located neighboring one another, the chambers (9) being separated by side walls (10) running in the longitudinal direction of the chambers (9), and having a height and length greater than its width, which Is defined by the distance of two neighboring side walls (10),
and wherein
the chambers (9) have two major sides (11) delimited by the side walls (10) and two long and two short minor sides (12, 13) extending between the side walls (10),
the chambers (9) contain an integrated sample acquisition and analyzing element, whose puncturing element (23) has a coupling structure (29) and is movable on the movement path of the puncture movement in the puncturing direction, and whose analyzing element (20) has a second coupling structure (31),
the chambers (9) have an exit opening (14) on one of their minor sides (12, 13) for allowing at least partial exiting out of the chamber (9) of a puncturing element (23) moved on the movement path,
the coupling unit (7) has a coupling element (33) and another coupling element (35), the chambers (9) of the magazine (3) are accessible for the coupling element (33) on one of their long minor sides (12) such that the coupling element (33) can be coupled to the coupling structure (29) of the puncturing element (23) and that the another coupling element (35) can be coupled to the coupling structure (31) of the analyzing element (20) when the puncturing element (23) and the analyzing element (20) are positioned in the chamber (9) of the magazine (3) and the magazine (3) is located in the holder.

2. Analysis system according to claim 1, **characterized in that** the measuring and evaluation unit (8) comprises an optical measuring apparatus for measuring an optically measurable measuring variable characteristic for the determination of the analyte.

3. Analysis system according to claim 1 or 2, **characterized in that** the analysis instrument comprises a contact pressure unit (36), by means of which the puncturing element (23) can be pressed against the analyzing element (20).

4. Analysis system according to claim 3, **characterized in that** the magazine (3) is advanced using the contact pressure unit (36).

5. Magazine for an analysis instrument, preferably as a component of an analysis system according to claim 1, having a plurality of flat, elongate, disposable integrated sample acquisition and analyzing elements, comprising a puncturing element (23) and an analyzing element (20) and having two flat sides,
wherein
the magazine (3) has a housing (4) including a plurality of elongate chambers (9) located neighboring one another,
the chambers (9) are separated by two side walls (10) running in the longitudinal direction of the chambers (9), and having a height and length is greater than the distance of two neighboring side walls (10),
the chambers (9) have two major sides (11) delimited by the side walls (10) and two long and two short minor sides (12, 13) extending between the side walls,
the chambers (9) contain an integrated sample acquisition and analyzing element, whose puncturing element (23) has a coupling structure (29) and is movable on a movement path of the puncture movement in the puncturing direction, and whose analyzing element (20) has a second coupling structure (31),
the chambers (9) have an exit opening (14) on one of their minor sides (12,13) for allowing at least partial exiting out of the chamber (9) of a puncturing element (23) moved on the movement path, and
the chambers (9) are accessible on one of their long minor sides (12) such that a coupling element (33) of a coupling unit (7) of the analysis instrument (2) can be coupled to the coupling structure (29) of the puncturing element (23) and that another coupling element (35) can be coupled to the coupling structure (31) of the analyzing element (20), wherein the puncturing element (23) and the analyzing element (20) are movable relative to another such that the puncturing element (23) is movable in a transfer position relative to the analyzing element (20) in which a transfer of a body fluid can take place.

6. Analysis system according to one of claims 1 to 4 or magazine according to claim 5, **characterized in that** the chambers (9) of the magazine (3) are covered with a film (17, 19) on the long minor side (12) on which the chambers (9) are accessible, and/or on the short minor side (13), on which the exit opening (14) of the chambers (9) is located.

7. Magazine for an analysis instrument according to claim 6,
wherein
the chambers (9) contain an integrated sample acquisition and analyzing element, whose puncturing element (23) is enclosed by a protective envelope (27),
and
the chambers (9) are covered with a film (17,19) on at least one of their sides (12) such that the chambers (9) are completely closed.

8. Magazine according to claim 7, **characterized in that**
the chambers are covered with a film on the long minor side (12) and/or on one of their minor sides (12,13) and/or on the short minor side (13) and/or on the major side (11).

9. Magazine according to claim 8, **characterized in that**
the chambers are accessible on one of their long minor side (12) such that a coupling element (33) of a coupling unit (7) of an analysis instrument (2) can be formfitting coupled to the coupling structure (29) of the puncturing element (23).

10. Analysis system or magazine according to any one of claims 6 to 9, **characterized in that** the film (17, 19) for covering the minor sides (12, 13) of the chambers (9) is at least partially metallic, and preferably consists of metal.

11. Analysis system or magazine according to any one of claim 6 to 10, **characterized In that** the film (17) which covers the chambers (9) on their long minor side can be penetrated by the coupling element (35) of the coupling unit (7) of the analysis instrument (2).

12. Analysis system or magazine according to any one of claims 6 to 11, **characterized in that** the film (19) which covers the exit opening (14) of the chambers (9) can be penetrated by the integrated sample acquisition and analyzing element (20) and/or by the puncturing element (23) on the movement path in the puncturing direction.

13. Analysis system or magazine according to any one of the preceding claims, **characterized in that** the coupling structure (29) of the puncturing element (23) and/or the second coupling structure (31) of the analyzing element (20) are located on the elongated minor side of the puncturing element (23) and/or the analyzing element (20), respectively, such that the coupling unit (7) of the analysis instrument (2) can be formfitting coupled to the coupling structure (29,31) and such that the puncturing element (23) and the analyzing element (22) are movable relative to another.

14. Analysis system or magazine according to any one of the preceding claims, **characterized in that**
the analyzing element (20) has a test field zone (21),
the puncturing element (23) has a capillary channel having at least one sample inlet and one sample outlet,
the movement path of the puncturing element (23) has a transfer position, in which the puncturing element (23) is located in relation to the analyzing element (20) in such a position that the sample outlet of the capillary channel of the puncturing element (23) neighbors the test field zone (21)) of the analyzing element (20) so that a transfer of a body fluid from the capillary channel onto the test field zone (21) can take place.

15. Analysis system or magazine according to any one of the preceding claims, **characterized In that** the Integrated sample acquisition and analyzing element comprises a spacer element (22), which is located between the analyzing element (20) and the puncturing element (23) such that the puncturing element (23) on its movement path at least in the puncturing direction passes a test field zone (21) of the analyzing element (20) without contacting the test field zone (21).

16. Analysis system or magazine according to any one of the preceding claims **characterized in that** the integrated sample acquisition and analyzing element (20) is movable in the puncturing direction into an operational position such that it at least partially extends to the exit opening (14) out of the chamber (9) of the magazine (3) and that a test field zone (21) of the analyzing element (20) is positioned outside the chamber (9) of the magazine (3).

17. Analysis system or magazine according to any one of the preceding claims,
**characterized in that**
- the puncturing element (23) is enclosed by a protective envelope (27),
- the protective envelope (27) is connected to the puncturing element (23) and to the analyzing element (20),
- the protective envelope (27) is telescoped upon a relative movement of the puncturing element (23) in relation to the analyzing element (20) in the puncturing direction, wherein the tip of the puncturing element (23) penetrates the protective envelope (27) and extends out of the protective envelope (27)

18. Method for analyzing a body fluid
using an analysis system (1), which comprises a reusable analysis instrument (2) and a magazine (3) including a plurality of flat, disposable, integrated sample acquisition and analyzing elements, each comprising a puncturing element (23) with a coupling structure (29) and an analyzing element (20) with a second coupling structure (31), and having two major surface,
the analysis Instrument (2) comprising a drive, a coupling unit (7) having a coupling element (33) for coupling to the coupling structure (29) of the puncturing element (23) and having a coupling element (35) for coupling to the coupling structure (31) of the analyzing element (20) of an integrated sample acquisition and analyzing element to the drive, a measuring and evaluation unit (8) for optical measurement of a measuring variable characteristic for the determination of an analyte and for deriving a desired analysis result, and a holder for holding the magazine (3),
the magazine (3) comprising a housing (4) having a plurality of elongate chambers (9) located neighboring one another,
the chambers (9) containing an integrated sample acquisition and analyzing element (20), whose analyzing element (20) has a test field zone (21) and a coupling structure (31), and the chambers (9) being accessible on one of their long minor sides such that the coupling element (35) of the coupling unit (7) of the analysis instrument (2) can be coupled to the analyzing element (20),
in particular using an analysis system (1) according to claim 1,
the method comprising the following steps:
a) coupling of the coupling element (35) to the coupling structure (31) of the analyzing element (20),
b) moving the analyzing element (20) in the puncturing direction into an operational position, in which the analyzing element (20) extends at least partially out of the chamber (9) of the magazine (3) such that the test field zone (21) of the analyzing element (20) is positioned outside the magazine (3),
c) placing a body fluid to be analyzed on the test field zone (21),
d) measuring a measurement variable, which is characteristic for the determination of an analyte, on the test field zone (21).

19. Method according to claim 18, wherein
the puncturing element (23) has a capillary channel having at least one sample inlet and one sample outlet,
**characterized by** the following further steps:
moving the puncturing element (23) synchronously to the analyzing element (20) in the puncturing direction until they reach the operational position together such that the test field zone (21) of the analyzing element (20) is positioned in front of the measuring and evaluation unit (8).

20. Method according to claim 19,
**characterized by** the following further steps:
e) moving the puncturing element (23) in relation to the analyzing element (20) on the movement path in the puncturing direction in such a position that the tip of the puncturing element (23) projects beyond the analyzing element (20) in the puncturing direction to receive a body fluid through the sample inlet in the capillary channel,
f) positioning the puncturing element (23) in relation to the analyzing element (20) in a transfer position in such a position that the sample outlet of the capillary channel of the puncturing element (23) neighbors the test field zone (21),
g) pressing the puncturing element (23) against the analyzing element (20) by means of a contact pressure unit (36) of the analysis instrument (2) such that the sample outlet of the capillary channel contacts the test field zone (21) and body fluid is transferred from the capillary channel onto the test field zone (21).

21. Method according to any one of claims 18 to 20, **characterized by** the following further step:
moving the integrated sample acquisition and analyzing element (20) opposite to the puncturing direction out of the operational position until the integrated sample acquisition and analyzing element (20) is positioned completely inside the chamber (9) of the magazine (3).

## Patentansprüche

1. Analysesystem zur Bestimmung eines Analyten in einer Körperflüssigkeit, umfassend
ein wiederverwendbares Analysegerät (2) und
ein Magazin (3) mit einer Mehrzahl von flachen disposiblen integrierten Probengewinnungs- und Analyseelementen, die jeweils ein Stechelement (23) und ein Analyseelement (20) einschließen und zwei Hauptoberflächen haben, wobei
das Analysegerät (2) umfasst
einen Antrieb, durch den eine Stechbewegung des Stechelements (23) angetrieben wird, um es auf einem Bewegungsweg während einer Vortriebsphase in Einstichrichtung und nach Erreichen eines Umkehrpunktes der Stechbewegung während einer Rückführungsphase entgegen der Einstichrichtung zu bewegen,
eine Kopplungseinheit (7), die dazu ausgebildet ist, ein integriertes Probengewinnungs- und Analyseelement mit dem Antrieb zu koppeln,
eine Mess- und Auswerteeinheit (8) zur Messung einer für die Bestimmung eines Analyten charakteristischen Messgröße und zur Ermittlung eines gewünschten Analyseergebnisses, und
eine Magazinhalterung zur Aufnahme des Magazins (3),
das Magazin (3) umfasst
eine Mehrzahl von nebeneinander angeordneten langgestreckten Kammern (9), die durch in Längsrichtung der Kammern (9) verlaufende Seitenwände (10) getrennt sind, mit einer Höhe und Länge, die größer sind als die Breite, die durch den Abstand zweier benachbarter Seitenwände (10) definiert ist,
und wobei
die Kammern (9) zwei von den Seitenwänden (10) begrenzte Hauptseiten (11) sowie je zwei sich zwischen den Seitenwänden (10) erstreckende lange und kurze Schmalseiten (12, 13) aufweisen,
die Kammern (9) je ein integriertes Probengewinnungs- und Analyseelement enthalten, dessen Stechelement (23) eine Mitnehmerstruktur (29) aufweist und auf dem Bewegungsweg der Stechbewegung in Einstichrichtung bewegbar ist, und dessen Analyseelement (20) eine zweite Mitnehmerstruktur (31) aufweist,
die Kammern (9) an einer ihrer Schmalseiten (12, 13) eine Austrittsöffnung (14) aufweisen, durch die ein auf dem Bewegungsweg bewegtes Stechelement (23) wenigstens teilweise aus der Kammer (9) heraustreten kann,
die Kopplungseinheit (7) ein Kopplungselement (33) und ein weiteres Kopplungselement (35) aufweist,
die Kammern (9) des Magazins (3) an einer ihrer langen Schmalseiten (12) für das Kopplungselement (33) derart zugänglich sind, dass das Kopplungselement (33) an die Mitnehmerstruktur (29) des Stechelements (23) ankoppelbar ist und dass das andere Kopplungselement (35) an die Mitnehmerstruktur (31) des Analyseelements (20) ankoppelbar ist, wenn das Stechelement (23) und das Analyseelement (20) in der Kammer (9) des Magazins (3) positioniert sind und das Magazin (3) in der Halterung angeordnet ist.

2. Analysesystem nach Anspruch 1, **dadurch gekennzeichnet, dass** die Mess- und Auswerteeinheit (8) eine optische Messeinrichtung umfasst, um eine optisch messbare, für die Bestimmung des Analyten charakteristische Messgröße zu messen.

3. Analysesystem nach Anspruch 1 oder 2, **dadurch gekennzeichnet, dass** das Analysegerät eine Andrückeinheit (36) umfasst, mit der das Stechelement (23) an das Analyseelement (20) andrückbar ist.

4. Analysesystem nach Anspruch 3, **dadurch gekennzeichnet, dass** das Magazin (3) mittels der Andrückeinheit (36) weitergetaktet wird.

5. Magazin für ein Analysegerät, vorzugsweise als Bestandteil eines Analysesystems nach Anspruch 1, mit einer Mehrzahl von flachen langgestreckten disposiblen integrierten Probengewinnungs- und Analyseelementen, die ein Stechelement (23) und ein Analyseelement (20) einschließen und zwei Flachseiten haben,
wobei
das Magazin (3) ein Gehäuse (4) mit einer Mehrzahl von nebeneinander angeordneten langgestreckten Kammern (9) hat,
die Kammern (9) durch zwei in Längsrichtung der Kammern (9) verlaufende Seitenwände (10) getrennt sind, und eine Höhe und Länge haben, die größer sind als der Abstand zweier benachbarter Seitenwände (10),
die Kammern (9) zwei von den Seitenwänden (10) begrenzte Hauptseiten (11) sowie je zwei sich zwischen den Seitenwänden erstreckende lange und kurze Schmalseiten (12, 13) aufweisen,
die Kammern (9) je ein integriertes Probengewinnungs- und Analyseelement enthalten, dessen Stechelement (23) eine Mitnehmerstruktur (29) aufweist und auf einem Bewegungsweg der Stechbewegung in Einstichrichtung bewegbar ist und dessen Analyseelement (20) eine zweite Mitnehmerstruktur (31) aufweist,
die Kammern (9) an einer ihrer Schmalseiten (12, 13) eine Austrittsöffnung (14) aufweisen, durch die ein auf dem Bewegungsweg bewegtes Stechelement (23) wenigstens teilweise aus der Kammer (9) heraustreten kann, und
die Kammern (9) an einer ihrer langen Schmalseiten (12) derart zugänglich sind, dass ein Kopplungselement (33) einer Kopplungseinheit (7) des Analysegeräts (2) an die Mitnehmerstruktur (29) des Stechelements (23) ankoppelbar ist und dass ein weiteres Kopplungselement (35) an die Mitnehmerstruktur (31) des Analyseelements (20) ankoppelbar ist, wobei das Stechelement (23) und das Analyseelement (20) derart relativ zueinander verschiebbar sind, dass das Stechelement (23) in einer Übertragungsposition, in der eine Übertragung einer Körperflüssigkeit stattfinden kann, relativ zu dem Analyseelement (20) verschiebbar ist.

6. Analysesystem nach einem der Ansprüche 1 bis 4 oder Magazin nach Anspruch 5, **dadurch gekennzeichnet, dass** die Kammern (9) des Magazins (3) an der langen Schmalseite (12), an der die Kammern (9) zugänglich sind, und/oder an der kurzen Schmalseite (13), an der sich die Austrittsöffnung (14) der Kammern (9) befindet, mit einer Folie (17, 18) abgedeckt sind.

7. Magazin für ein Analysegerät nach Anspruch 5, wobei die Kammern (9) ein integriertes Probengewinnungs- und Analyseelement enthalten, dessen Stechelement (23) von einer Schutzhülle (27) umschlossen ist,
und
die Kammern (9) auf mindestens einen ihrer Seiten (12) derart von einer Folie (17, 19) abgedeckt sind, dass die Kammern (9) komplett verschlossen sind.

8. Magazin nach Anspruch 7, **dadurch gekennzeichnet dass** die Kammern an der langen Schmalseite (12) und/oder an einer ihrer Schmalseiten (12, 13) und/oder an der kurzen Schmalseite (13) und/oder an der Hauptseite (11) von einer Folie abgedeckt sind.

9. Magazin nach Anspruch 8, **dadurch gekennzeichnet, dass** die Kammern an einer ihrer langen Schmalseiten (12) derart zugänglich sind, dass ein Kopplungselement (33) einer Kopplungseinheit (7) eines Analysegeräts (2) formschlüssig an die Mitnehmerstruktur (29) des Stechelements (23) ankoppelbar ist.

10. Analysesystem oder Magazin nach einem der Ansprüche 6 bis 9, **dadurch gekennzeichnet, dass** die Folie (17, 19) zur Abdeckung der Schmalseiten (12, 13) der Kammern (9) wenigstens teilweise metallisch ist, bevorzugt aus Metall besteht.

11. Analysesystem oder Magazin nach einem der Ansprüche 6 bis 10, **dadurch gekennzeichnet, dass** die Folie (17), die die Kammern (9) an ihren langen Schmalseiten abdeckt, mittels des Kopplungselements (35) der Kopplungseinheit (7) des Analysegerätes (2) durchstoßbar ist.

12. Analysesystem oder Magazin nach einem der Ansprüche 6 bis 11, **dadurch gekennzeichnet, dass** die Folie (19), die die Austrittsöffnung (14) der Kammern (9) abdeckt, von dem integrierten Probengewinnungs- und Analyseelement (20) und/oder von dem Stechelement (23) auf dem Bewegungsweg in Einstichrichtung durchstoßbar ist.

13. Analysesystem oder Magazin nach einem vorhergehenden Ansprüche, **dadurch gekennzeichnet, dass** die Mitnehmerstruktur (29) des Stechelements (23) und/oder die zweite Mitnehmerstruktur (31) des Analyseelements (20) derart an der langgestreckten Schmalseite des Stechelements (23) bzw. des Analyseelements (20) angebracht sind, dass die Kopplungseinheit (7) des Analysegeräts (2) formschlüssig an die Mitnehmerstruktur (29, 31) ankoppelbar ist und dass das Stechelement (23) und das Analyseelements (20) relativ zueinander verschiebbar sind.

14. Analysesystem oder Magazin nach einem der vorhergehenden Ansprüche, **dadurch gekennzeichnet, dass**
das Analyseelement (20) eine Testfeldzone (21) aufweist,
das Stechelement (23) einen Kapillarkanal mit wenigstens einem Probeneinlass und einem Probenauslass hat,
der Bewegungsweg des Stechelements (23) eine Übergabeposition aufweist, in der das Stechelement (23) relativ zu dem Analyseelement (20) derart angeordnet ist, dass der Probenauslass des Kapillarkanals des Stechelements (23) der Testfeldzone (21) des Analyseelements (20) so benachbart ist, dass eine Übertragung einer Körperflüssigkeit aus dem Kapillarkanal auf die Testfeldzone (21) stattfinden kann.

15. Analysesystem oder Magazin nach einem der vorhergehenden Ansprüche, **dadurch gekennzeichnet, dass** das integrierte Probengewinnungs- und Analyseelement ein Distanzelement (22) umfasst, dass derart zwischen dem Analyseelement (20) und dem Stechelement (23) angeordnet ist, dass das Stechelement (23) auf seinem Bewegungsweg wenigstens in Einstichrichtung eine Testfeldzone (21) des Analyseelements (20) passiert, ohne diese zu berühren.

16. Analysesystem oder Magazin nach einem der vorhergehenden Ansprüche, **dadurch gekennzeichnet, dass** das integrierte Probengewinnungs- und Analyseelement (20) in Einstichrichtung in eine Arbeitsposition derart bewegbar ist, dass es sich wenigstens teilweise durch die Austrittsöffnung (14) aus der Kammer (9) des Magazins (3) hinaus erstreckt und dass eine Testfeldzone (21) des Analyseelements (20) außerhalb der Kammer (9) des Magazins (3) positioniert ist.

17. Analysesystem oder Magazin nach einem der vorhergehenden Ansprüche, **dadurch gekennzeichnet, dass**
- das Stechelement (23) von einer Schutzhülle (27) umschlossen ist,
- die Schutzhülle (27) mit dem Stechelement (23) und dem Analyseelement (20) verbunden ist,
- die Schutzhülle (27) bei einer Relativbewegung des Stechelements (23) relativ zu dem Analyseelement (20) in Einstichrichtung zusammengeschoben wird, wobei die Spitze des Stechelements (23) die Schutzhülle (27) durchsticht und sich aus der Schutzhülle (27) hinaus erstreckt.

18. Verfahren zur Untersuchung einer Körperflüssigkeit mittels eines Analysesystems (1), welches ein wiederverwendbares Analysegerät (2) und ein Magazin (3) mit einer Mehrzahl von flachen disposiblen integrierten Probengewinnungs- und Analyseelementen, die jeweils ein Stechelement (23) mit einer Mitnehmerstruktur (29) und ein Analyseelement (20) mit einer zweiten Mitnehmerstruktur (31) einschließen und zwei Hauptoberflächen haben,
wobei
das Analysegerät (2) einen Antrieb und eine Kopplungseinheit (7) mit einem Kopplungselement (33) zur Kopplung mit der Mitnehmerstruktur (29) des Stechelements (23) sowie mit einem Kopplungselement (35) zur Kopplung mit der Mitnehmerstruktur (31) des Analyseelements (20), um das integrierte Probengewinnungs- und Analyseelement mit dem Antrieb zu koppeln, sowie eine Mess- und Auswerteeinheit (8) zur optischen Messung einer für die Bestimmung eines Analyten charakteristischen Messgröße und zur Ermittlung eines gewünschten Analyseergebnisses, und eine Halterung zur Aufnahme des Magazins (3) umfasst,
das Magazin (3) ein Gehäuse (4) mit einer Mehrzahl von nebeneinander angeordneten langgestreckten Kammern (9), umfasst wobei die Kammern (9) je ein integriertes Probengewinnungs- und Analyseelement (20) enthalten, dessen Analyseelement (20) eine Testfeldzone (21) und eine Mitnehmerstruktur (31) hat, und die Kammern (9) an einer ihrer langen Schmalseiten derart zugänglich sind, dass das Kopplungselement (35) der Kopplungseinheit (7) des Analysegeräts (2) an das Analyseelement (20) ankoppelbar ist,
insbesondere mittels eines Analysesystems (1) nach Anspruch 1, wobei das Verfahren die folgenden Schritte umfasst:
a) Ankoppeln des Kopplungselements (35) an die Mitnehmerstruktur (31) des Analyseelements (20),
b) Bewegen des Analyseelements (20) in Einstichrichtung in eine Arbeitsposition, in der sich das Analyseelement (20) mindestens teilweise derart aus der Kammer (9) des Magazins (3) hinaus erstreckt, dass die Testfeldzone (21) des Analyseelements (20) außerhalb des Magazins (3) positioniert ist,
c) Platzieren einer zu analysierenden Körperflüssigkeit auf der Testfeldzone (21),
d) Messen einer Messgröße, die für die Bestimmung eines Analyten auf der Testfeldzone (21) charakteristisch ist.

19. Verfahren nach Anspruch 18, wobei
das Stechelement (23) einen Kapillarkanal mit wenigstens einem Probeneinlass und einem Probenauslass aufweist,
**gekennzeichnet durch** die folgenden weiteren Schritte:
Bewegen des Stechelements (23) synchron zu dem Analyseelement (20) in Einstichrichtung, bis sie gemeinsam die Arbeitsposition erreichen, sodass sich die Testfeldzone (21) des Analyseelements (20) vor der Mess- und Auswerteeinheit (8) befindet.

20. Verfahren nach Anspruch 19,
**gekennzeichnet durch** die folgenden weiteren Schritte: e) Bewegen des Stechelements (23) relativ zu dem Analyseelement (20) auf dem Bewegungsweg in Einstichrichtung derart, dass die Spitze des Stechelements (23) das Analyseelement (20) in Einstichrichtung überragt, um eine eine Körperflüssigkeit **durch** den Probeneinlass in den Kapillarkanal aufzunehmen,
f) Positionieren des Stechelements (23) relativ zu dem Analyseelement (20) in einer Übergabeposition derart, dass der Probenauslass des Kapillarkanals des Stechelements (23) der Testfeldzone (21) benachbart ist,
g) Andrücken des Stechelements (23) an das Analyseelement (20) mittels einer Andrückeinheit (36) des Analysegeräts (2) derart, dass der Probenauslass des Kapillarkanals an die Testfeldzone (21) gedrückt und Körperflüssigkeit aus dem Kapillarkanal auf die Testfeldzone (21) übertragen wird.

21. Verfahren nach einem der Ansprüche 18 bis 20, **gekennzeichnet durch** den folgenden weiteren Schritt:
Bewegen des integrierten Probengewinnungs- und Analyseelements (20) aus der Arbeitsposition entgegen der Einstichrichtung, bis das integrierte Probengewinnungs- und Analyseelement (20) vollständig in der Kammer (9) des Magazins (3) positioniert ist.

## Revendications

1. Système d'analyse pour déterminer un analyte dans un fluide corporel, le système comprenant
un instrument (2) d'analyse réutilisable et
un magasin (3) incluant une pluralité d'éléments plats, jetables et intégrés d'acquisition et d'analyse d'échantillons, comprenant chacun un élément (23) de perforation et un élément (20) d'analyse et ayant deux surfaces majeures,
l'instrument d'analyse (2) comprenant
un entraînement, par lequel un mouvement de perforation de l'élément (23) de perforation est entraîné sur un chemin de mouvement, le mouvement de perforation incluant une phase de propulsion dans le sens de perforation et, après atteinte d'un point de retour, une phase de retrait opposée au sens de perforation,
une unité (7) de couplage, adaptée à coupler un élément intégré
d'acquisition et d'analyse d'échantillons à l'entraînement,
une unité (8) de mesure et d'évaluation pour mesurer une variable de
mesure caractéristique pour la détermination d'un analyte et pour dériver de celle-ci un résultat d'analyse désiré, et
un porte-magasin pour retenir le magasin (3),
le magasin (3) comprenant
une pluralité de chambres (9) allongées situées voisines les unes des
autres, les chambres (9) étant séparées par des parois latérales (10) courant dans le sens longitudinal des chambres (9), et ayant une hauteur et une longueur supérieures à leur largeur, qui est définie par la distance de deux parois latérales (10) voisines,
et dans lequel
les chambres (9) ont deux côtés majeurs (11) délimités par les parois latérales (10) et deux côtés mineurs longs et deux courts (12, 13) s'étendant entre les parois latérales (10),
les chambres (9) contiennent un élément intégré d'acquisition et d'analyse d'échantillons, dont l'élément (23) de perforation a une structure (29) de couplage et est mobile sur le chemin de mouvement du mouvement de perforation dans le sens de perforation, et dont l'élément (20) d'analyse a une deuxième structure (31) de couplage,
les chambres (9) ont une ouverture (14) de sortie sur un de leurs côtés mineurs (12, 13) pour permettre une sortie au moins partielle hors de la chambre (9) d'un élément (23) de perforation déplacé sur le chemin de mouvement,
l'unité (7) de couplage a un élément (33) de couplage et un autre élément (35) de couplage,
les chambres (9) du magasin (3) sont accessibles pour l'élément (33) de couplage sur un de leurs côtés mineurs longs (12) de telle sorte que l'élément (33) de couplage peut être couplé à la structure (29) de couplage de l'élément (23) de perforation et que l'autre élément (35) de couplage peut être couplé à la structure (31) de couplage de l'élément (20) d'analyse lorsque l'élément (23) de perforation et l'élément (20) d'analyse sont positionnés dans la chambre (9) du magasin (3) et que le magasin (3) est situé dans le porte-magasin.

2. Système d'analyse selon la revendication 1, **caractérisé en ce que** l'unité (8) de mesure et d'évaluation comprend un appareil de mesure optique pour mesurer une variable de mesure optiquement mesurable pour la détermination de l'analyte.

3. Système d'analyse selon la revendication 1 ou 2, **caractérisé en ce que** l'instrument d'analyse comprend une unité (36) de pression de contact, au moyen de laquelle l'élément (23) de perforation peut être pressé contre l'élément (20) d'analyse.

4. Système d'analyse selon la revendication 3, **caractérisé en ce que** le magasin (3) est avancé en utilisant l'unité (36) de pression de contact.

5. Magasin pour un instrument d'analyse, préférablement comme un composant d'un système d'analyse selon la revendication 1, ayant une pluralité d'éléments plats, jetables et intégrés d'acquisition et d'analyse d'échantillons, comprenant un élément (23) de perforation et un élément (20) d'analyse et ayant deux côtés plats,
dans lequel
le magasin (3) a un logement (4) incluant une pluralité de chambres (9) allongées situées voisines les unes des autres,
les chambres (9) sont séparées par deux parois latérales (10) courant dans le sens longitudinal des chambres (9), et ayant une hauteur et une longueur supérieures à la distance de deux parois latérales (10) voisines,
les chambres (9) ont deux côtés majeurs (11) délimités par les parois latérales (10) et deux côtés mineurs longs et deux courts (12, 13) s'étendant entre les parois latérales,
les chambres (9) contiennent un élément intégré d'acquisition et d'analyse d'échantillons, dont l'élément (23) de perforation a une structure (29) de couplage et est mobile sur le chemin de mouvement du mouvement de perforation dans le sens de perforation, et dont l'élément (20) d'analyse a une deuxième structure (31) de couplage,
les chambres (9) ont une ouverture (14) de sortie sur un de leurs côtés mineurs (12, 13) pour permettre une sortie au moins partielle hors de la chambre (9) d'un élément (23) de perforation déplacé sur le chemin de mouvement, et
les chambres (9) sont accessibles sur un de leurs côtés mineurs longs (12) de telle sorte qu'un élément (33) de couplage d'une unité (7) de couplage de l'instrument (2) d'analyse peut être couplé à la structure (29) de couplage de l'élément (23) de perforation et qu'un autre élément (35) de couplage peut être couplé à la structure (31) de couplage de l'élément (20) d'analyse, dans lequel l'élément (23) de perforation et l'élément (20) d'analyse sont mobiles l'un par rapport à l'autre de telle sorte que l'élément (23) de perforation peut être déplacé dans une position de transfert par rapport à l'élément (20) d'analyse dans lequel un transfert d'un fluide corporel peut avoir lieu.

6. Système d'analyse selon l'une des revendications 1 à 4 ou magasin selon la revendication 5, **caractérisé en ce que** les chambres (9) du magasin (3) sont recouvertes avec un film (17, 19) sur le côté mineur long (12) sur lequel les chambres (9) sont accessibles, et/ou sur le côté mineur court (13), sur lequel l'ouverture (14) de sortie des chambres (9) est située.

7. Magasin pour un instrument d'analyse selon la revendication 5, dans lequel
les chambres (9) contiennent un élément intégré d'acquisition et d'analyse d'échantillons, dont l'élément (23) de perforation est renfermé par une enveloppe (27) de protection,
et
les chambres (9) sont recouvertes avec un film (17, 19) sur au moins un de leurs côtés (12), de telle sorte que les chambres (9) sont totalement fermées.

8. Magasin selon la revendication 7, **caractérisé en ce que** les chambres sont recouvertes avec un film sur le côté mineur long (12) et/ou sur un de leurs côtés mineurs (12, 13) et/ou sur le côté mineur court (13) et/ou sur le côté majeur (11).

9. Magasin selon la revendication 8, **caractérisé en ce que** les chambres sont accessibles sur un de leur côté mineur long (12) de telle sorte qu'un élément (33) de couplage d'une unité (7) de couplage d'un instrument (2) d'analyse peut être couplé par conjugaison de forme à la structure (29) de couplage de l'élément (23) de perforation.

10. Système d'analyse ou magasin selon l'une quelconque des revendications 6 à 9, **caractérisé en ce que** le film (17, 19) pour recouvrir les côtés mineurs (12, 13) des chambres (9) est au moins partiellement métallique, et préférablement consiste en un métal.

11. Système d'analyse ou magasin selon l'une quelconque des revendications 6 à 10, **caractérisé en ce que** le film (17) qui recouvre les chambres (9) sur leur côté mineur long peut être pénétré par l'élément (35) de couplage de l'unité (7) de couplage de l'instrument (2) d'analyse.

12. Système d'analyse ou magasin selon l'une quelconque des revendications 6 à 11, **caractérisé en ce que** le film (19) qui recouvre l'ouverture (4) de sortie des chambres (9) peut être pénétré par l'élément (20) intégré d'acquisition et d'analyse d'échantillons et/ou par l'élément (23) de perforation sur le chemin de mouvement dans le sens de perforation.

13. Système d'analyse ou magasin selon l'une quelconque des revendications précédentes, **caractérisé en ce que** la structure (29) de couplage de l'élément (23) de perforation et/ou la deuxième structure (31) de couplage de l'élément (20) d'analyse sont situées sur le côté mineur allongé de l'élément (23) de perforation et/ou de l'élément (20) d'analyse, respectivement, de telle sorte que l'unité (7) de couplage de l'instrument (2) d'analyse peut être couplée par conjugaison de forme à la structure (29, 31) de couplage et de telle sorte que l'élément (23) de perforation et l'élément (22) d'analyse sont mobiles l'un par rapport à l'autre.

14. Système d'analyse ou magasin selon l'une quelconque des revendications précédentes, **caractérisé en ce que**
l'élément (20) d'analyse a une zone (21) de champ de test,
l'élément (23) de perforation a un canal capillaire ayant au moins une entrée d'échantillon et une sortie d'échantillon,
le chemin de mouvement de l'élément (23) de perforation a une position de transfert, dans laquelle l'élément (23) de perforation est situé en relation à l'élément (20) d'analyse dans une position telle que la sortie d'échantillon du canal capillaire de l'élément (23) de perforation est voisine de la zone (21) de champ de test de l'élément (20) d'analyse de telle sorte qu'un transfert d'un fluide corporel du canal capillaire jusque sur la zone (21) de champ de test peut avoir lieu.

15. Système d'analyse ou magasin selon l'une quelconque des revendications précédentes, **caractérisé en ce que** l'élément intégré d'acquisition et d'analyse d'échantillons comprend un élément (22) espaceur, qui est situé entre l'élément (20) d'analyse et l'élément (23) de perforation de telle sorte que l'élément (23) de perforation sur son chemin de mouvement au moins dans le sens de perforation passe au-delà d'une zone (21) de champ de test de l'élément (20) d'analyse sans entrer en contact avec la zone (21) de champ de test.

16. Système d'analyse ou magasin selon l'une quelconque des revendications précédentes, **caractérisé en ce que** l'élément (20) intégré d'acquisition et d'analyse d'échantillons est mobile dans le sens de perforation jusque dans une position opérationnelle de telle sorte qu'il s'étend au moins partiellement jusqu'à l'ouverture (14) de sortie hors de la chambre (9) du magasin (3) et qu'une zone (21) de champ de test de l'élément (20) d'analyse est positionnée à l'extérieur de la chambre (9) du magasin (3).

17. Système d'analyse ou magasin selon l'une quelconque des revendications précédentes, **caractérisé en ce que**
- l'élément (23) de perforation est renfermé par une enveloppe (27) de protection,
- l'enveloppe (27) de protection est connectée à l'élément (23) de perforation et à l'élément (20) d'analyse,
- l'enveloppe (27) de protection est télescopée lors d'un mouvement relatif de l'élément (23) de perforation par rapport à l'élément (20) d'analyse dans le sens de perforation, dans lequel la pointe de l'élément (23) de perforation pénètre l'enveloppe (27) de protection et s'étend hors de l'enveloppe (27) de protection.

18. Procédé pour analyser un fluide corporel
utilisant un système (1) d'analyse, qui comprend un instrument (2) d'analyse réutilisable et un magasin (3) incluant une pluralité d'éléments plats, jetables et intégrés d'acquisition et d'analyse d'échantillons,
comprenant chacun un élément (23) de perforation avec une structure (29) de couplage et un élément (20) d'analyse avec une deuxième structure (31) de couplage, et ayant deux surfaces majeures, l'instrument (2) d'analyse comprenant un entraînement, une unité (7) de couplage ayant un élément (33) de couplage pour un couplage à la structure (29) de couplage de l'élément (23) de perforation et ayant un élément (35) de couplage pour un couplage à la structure (31) de couplage de l'élément (20) d'analyse d'un élément intégré d'acquisition et d'analyse d'échantillons à l'entraînement, une unité (8) de mesure et d'évaluation pour une mesure optique d'une variable de mesure caractéristique pour la détermination d'un analyte et pour dériver un résultat d'analyse désiré, et un porte-magasin pour retenir le magasin (3),
le magasin (3) comprenant un logement (4) ayant une pluralité de chambres (9) allongées situées voisines les unes des autres,
les chambres (9) contenant un élément (20) intégré d'acquisition et d'analyse d'échantillons, dont l'élément (20) d'analyse a une zone (21) de champ de test et une structure (31) de couplage, et les chambres (9) étant accessibles sur un de leurs côtés mineur long de telle sorte que l'élément (35) de couplage de l'unité (7) de couplage de l'instrument (2) d'analyse peut être couplé à l'élément (20) d'analyse,
en particulier utilisant un système (1) d'analyse selon la revendication 1,
le procédé comprenant les étapes suivantes :
a) couplage de l'élément (35) de couplage à la structure (31) de couplage de l'élément (20) d'analyse,
b) déplacement de l'élément (20) d'analyse dans le sens de perforation jusque dans une position opérationnelle, dans laquelle l'élément (20) d'analyse s'étend au moins partiellement hors de la chambre (9) du magasin (3) de telle sorte que la zone (21) de champ de test de l'élément (20) d'analyse est positionnée à l'extérieur du magasin (3),
c) placement d'un fluide corporel à analyser sur la zone (21) de champ de test,
d) mesure d'une variable de mesure, qui est caractéristique pour la détermination d'un analyte, sur la zone (21) de champ de test.

19. Procédé selon la revendication 18, dans lequel l'élément (23) de perforation a un canal capillaire ayant au moins une entrée d'échantillon et une sortie d'échantillon,
**caractérisé par** les autres étapes suivantes :
déplacement de l'élément (23) de perforation de façon synchrone avec l'élément (20) d'analyse dans le sens de perforation jusqu'à ce qu'ils atteignent la position opérationnelle ensemble de telle sorte que la zone (21) de champ de test de l'élément (20) d'analyse est positionnée en avant de l'unité (8) de mesure et d'évaluation.

20. Procédé selon la revendication 19,
**caractérisé par** les autres étapes suivantes :
e) déplacement de l'élément (23) de perforation par rapport à l'élément (20) d'analyse sur le chemin de mouvement dans le sens de perforation dans une position telle que la pointe de l'élément (23) de perforation se projette au-delà de l'élément (20) d'analyse dans le sens de perforation pour recevoir un fluide corporel à travers l'entrée d'échantillon dans le canal capillaire,
f) positionnement de l'élément (23) de perforation par rapport à l'élément (20) d'analyse dans une position de transfert dans une position telle que la sortie d'échantillon du canal capillaire de l'élément (23) de perforation est voisine de la zone (21) de champ de test,
g) pressage de l'élément (23) de perforation contre l'élément (20) d'analyse au moyen d'une unité (36) de pression de contact de l'instrument (2) d'analyse de telle sorte que la sortie d'échantillon du canal capillaire vient en contact avec la zone (21) de champ de test et qu'un fluide corporel est transféré du canal capillaire jusque sur la zone (21) de champ de test.

21. Procédé selon l'une quelconque des revendications 18 à 20, **caractérisé par** l'autre étape suivante :
déplacement de l'élément (20) intégré d'acquisition et d'analyse d'échantillons dans le sens opposé au sens de perforation hors de la position opérationnelle jusqu'à ce que l'élément (20) intégré d'acquisition et d'analyse d'échantillons soit positionné complètement à l'intérieur de la chambre (9) du magasin (3).
